# EUROPEAN PATENT APPLICATION

(11) **EP 3 473 235 A1**
(43) Date of publication of application: **24.04.2019**
(21) Application number: 17740040.5
(22) Date of filing: 19.06.2017
(51) Int. Cl.: A61K 8/24, A61K 8/25, A61Q 11/00

(54) **COMPOSITION OF MATERIALS FOR TOOTH REMINERALISATION**

(30) Priority: 20.06.2016 ES 201630835
(71) Applicant: Helicon Medical, S.L., 28760 Tres Cantos (Madrid) (ES)
(72) Inventor: GARCÍA DE CASTRO ANDREWS, Arcadio, 28760 Tres Cantos (Madrid) (ES); GARCÍA CARRODEGUAS, Raúl, 28760 Tres Cantos (Madrid) (ES); PADILLA MONDEJAR, Sussette, 28760 Tres Cantos (Madrid) (ES); GARZÓN GUTIÉRREZ, Ana, 28760 Tres Cantos (Madrid) (ES); TOLEDANO PÉREZ, Manuel, 18071 Granada (ES); OSORIO RUIZ, Raquel, 18071 Granada (ES)
(74) Representative: Pons Ariño, Angel
(86) International application number: PCT/ES2017/070444
(87) International publication number: WO 2017/220835

(57) **Abstract**

The present invention relates to materials comprising a combination of calcium phosphates and silicon compounds without calcium, and which show unexpected efficiency in tooth re-mineralisation and occlusion of exposed dentinal tubules when used as a single component or in dental products for oral hygiene and treatment. The preferred calcium phosphates include anhydrous dicalcium phosphate, dihydrate dicalcium phosphate, hydroxylapatite and amorphous calcium phosphate, these phosphates being able to incorporate partial substitutions of calcium by divalent metallic ions such as Mg++, Zn++, Ba++, Fe++, Sn++ o Sr++. The preferred silicon compounds include silicon dioxide, silica gel, metasilicic acid, orthosilicic acid, silicic acid and combinations thereof..

## Description

### FIELD OF INVENTION

Present invention relates to materials with the capacity to re-mineralize teeth comprising calcium phosphates and calcium-free silicon compounds and their application in the formulation of dentifrice formulations.

### BACKGROUND ART

Teeth have an internal dentin layer and an outer hard enamel layer composed of tightly packed aligned hydroxyapatite crystals. In humans, enamel varies in thickness over the surface of the tooth, often thickest at the cusp, up to 2.5 mm, and thinnest at its border with the cementum at the cement-enamel junction. The formation and destruction of enamel hydroxyapatite on the tooth surface is under a continuous dynamic process in which hydroxyapatite dissolves and re-precipitates on surface of teeth. However, tooth de-mineralization processes may occur as a result of the alteration of this balance favored by an acid environment in which hydroxyapatite is more soluble. This acid environment may result directly from diet or indirectly by lactic acid produced by bacterial fermentation of dietary sugars. The use of fluoride in dental products has proved to be an effective measure to reduce tooth caries as it incorporates into apatite to produce fluorapatite that is more resistant to exposure to acid environments.

Loss of tooth enamel, dental cementum and soft tissue surrounding a teeth, result in exposure of dentin that is thereafter subject to further physical and chemical challenges that lead to further loss of dental tissue. Exposed dentinal tubules result in dentin hypersensitivity and increased exposure to further bacterial attack. Desensitizing dentifrices with potassium oxalate have been found to provide temporary tubule occlusion however, treatment of de-mineralized teeth and exposed dentin should aim at prolonged occlusion and re-mineralization of tubules and inter-tubular dentin.

Modern approaches towards preventing tooth decay aim at re-mineralizing dentin. An example of this is set in U.S. Pat. No. 5,268,167 and U.S. Pat. No. 5,037,639 that disclose the use of amorphous calcium compounds such as amorphous calcium phosphate, amorphous calcium phosphate fluoride and amorphous calcium carbonate phosphate for use in re-mineralizing teeth by promoting the formation of a protective fluorapatite layer. US Pat. No. 5,437,857 also discloses use of amorphous calcium compounds such as amorphous calcium phosphate (ACP), amorphous calcium phosphate fluoride (ACPF), amorphous calcium carbonate phosphate (ACCP), amorphous calcium carbonate phosphate fluoride (ACCPF), and amorphous calcium fluoride (ACF) for use in re-mineralizing and fluoridating teeth. Inventors claim high solubility but this may be disadvantageous for prolonged deposition and in sequestering fluoride in the formulation. In another example, U.S. Pat. No. 5,427,768 describe calcium phosphate solutions that are supersaturated with respect to calcium phosphate solids and carbon dioxide. Patent claims that these solutions deposit calcium phosphate compounds with or without fluoride on and in the tooth weaknesses such as dental caries, exposed root, or dentin.

Patent EP2626058 describes a kit comprising a combination of poorly soluble calcium phosphate particles with phosphate-free calcium compounds that when mixed together with water react to form hydroxyapatite according to the cementing reactions described in U.S. Pat. No. 5,782,971. This prior art has the main disadvantage that product needs to be premixed with water by the user for the cementing reaction to occur. Another example in the approach to re-mineralize dentin is provided by the use of bioactive amorphous glasses, composed of SiO₂, Na₂O, CaO and P₂O₅ and obtained by high temperature treatments, commonly named as 45S5.

International patent publication WO96/10985 discloses particles of bioactive glasses containing silica that are exemplified by a glass of composition SiO₂ 45%; CaO 22%; P₂O₅ 78%; Na₂O 24% and B₂O₃ 2%, that can reduce pulpal irritation of a tooth and/or strengthen the structure of a tooth, and therefore have use in the treatment of hypersensitive teeth. It is suggested that such glasses can consist solely of silicon oxide or silicon hydroxide or can contain one or more additional elements selected from Ca, P, Na, K, Al, B, N, Mg, Ti, or F. It is also suggested that it is advisable to use bioactive glass compositions comprising calcium and phosphate which can help induce re-mineralization of dentin or alternatively to use separate sources of calcium and phosphate together with a bioactive glass not containing them.

U.S. Patents No. 6,086,374 and No. 5,735,942 disclose the use for prevention and treatment of tooth decay, re-mineralizing enamel, dentin hypersensitivity and occluding dentinal tubules, of bioactive glasses with the following composition SiO₂ 40-60%, CaO 10-30 %, Na₂O 10-35%, P₂O₅ 2-8%, CaF2 0-25% , B₂O₃ 0-10%, K₂O 0-8% and MgO 0-5%. Bioactive glasses are normally used as particles less than 90 µm with a an effective re-mineralizing amount of particles less than about 10 µm. Inventors indicate that the use of bioactive glass particles in these size ranges produce a stable crystalline carbonate apatite layer deposited onto and into the dentin tubules to obtain the desired effects. International patent publications WO 97/27148 and WO 99/13852 also relate to the use of bioactive glasses for re-mineralization of dentin.

Incorporation of bioactive glasses in dentifrices is exemplified in international patent publication WO 2005/063185 that describes the incorporation of NovaMin® bioglass, which is identified as 45S5 Bioglass® with a composition about 45% silicon dioxide, about 24.5% sodium oxide, 6% phosphorus oxide, and 24.5% calcium oxide. Inventors describe non-aqueous compositions of carboxyvinyl polymer, a humectant, a polyethylene glycol and about 0.25% to about 10%, preferably between 2% and 5% of bioactive glass particles having an average particle size of less than about 20 µm, ideally about 2 µm.

More recently international patent publication WO 2013/034421 describes dentifrice compositions that contain calcium sources such as hydroxyapatite, amorphous calcium phosphate or calcium silicates in combination with organic acids such as glutamic acid, aspartic acid and glycine and sodium and potassium phosphates as phosphate sources.

Although with a different purpose, the field of bone regeneration also makes use of synthetic biomaterials based on calcium phosphates and silicates to promote the restoration of bone defects. Many alternative formulations have been proposed for bone regeneration although hydroxyapatite and tricalcium phosphate are the most frequently used in the clinic. More recently, international patent publication WO 2010/094813 describes bone regeneration materials based on combinations of monetite with other bioactive calcium and silicon compounds. However, bone repair is a biological process that is fundamentally different to the physicochemical processes that occur during teeth re-mineralization. Bone repair involves active participation of cells involved in bone remodeling, osteoblasts and osteoclasts and takes place over many months during which osteoblasts lay down new self-bone. Alternatively, treatments for re-mineralization of dental tissues involve physical occlusion of exposed dentinal tubes and precipitation of calcium phosphates on the tooth surface within the few minutes during daily tooth brushing. Therefore, there can be no inference that a bone repair material will necessarily prove useful or applicable in tooth re-mineralization or vice versa. Consequent with this, there is no mention in international patent publication WO 2010/094813 that the materials disclosed for bone regeneration may be of use for tooth re-mineralization.

Present invention provides with an improved materials for prevention and treatment of tooth decay, re-mineralizing dentin and enamel, occluding dentinal tubules, and treatment of dentin hypersensitivity, which results from combining calcium phosphates with calcium-free silicon compounds. Present invention takes advantage of established *in vitro* models to demonstrate significant improvement of the present materials over established re-mineralizing materials such as 45S5 bioactive glass claimed in Novamin®. Present invention also relates to the incorporation of these materials in dentifrices and their use for re-mineralization of teeth, prevention of caries, re-mineralization of dentin and enamel, occlusion of dentinal tubules and treatment of tooth hypersensitivity.

### DETAILED DESCRIPTION

Teeth are mineralized tissues continuously exposed to chemical and physical damage. A main process of tooth damage is by de-mineralization resulting from dissolution of the hydroxyapatite that forms tooth enamel and cement. Comparative re-mineralization studies carried out in explanted teeth treated to remove enamel and expose dentin have resulted in the identification of combination materials of the present invention containing calcium phosphates and calcium-free silicon compounds that are efficacious in re-mineralizing teeth. According to a first embodiment of the present invention there is provided materials for tooth re-mineralization, referred to also as re-mineralizing materials, comprising between 50% and 95% in mass of a calcium phosphate and between 1% and 40% in mass of a calcium-free silicon compound. Daily tooth brushing with dentifrices that contain the re-mineralizing materials of the present invention result in the occlusion of dentinal tubules, tooth re-mineralization and reduction of dentinal hypersensitivity.

Comparative studies incorporated as Examples in the present invention demonstrate that the re-mineralizing materials of the present invention provide with improved re-mineralization of teeth when compared to Bioglass 45S5 (Novamin®). Materials of the present invention have been found to have an excellent capacity to obstruct exposed dentinal tubules, re-mineralize dentin, and form apatite on the surface of teeth by exposure during normal daily tooth brushing. Furthermore, dentin re-mineralized with the materials of the present invention have been found to be more resistant to a subsequent acid attack that simulates conditions normally encountered by teeth in the oral cavity. Preferred materials include combinations of calcium-free silicon compounds with calcium phosphates are selected from: anhydrous dicalcium phosphate [monetite, CaHPO₄], dicalcium phosphate dihydrate [brushite, CaHPO₄·2H₂O], hydroxyapatite [Ca₁₀(PO₄)₆(OH)₂], amorphous calcium phosphate [Ca₃(PO₄)₂·nH₂O], and combinations thereof. Therefore, according to a second embodiment of the present invention there is provided materials for tooth re-mineralization comprising between 1% and 40% in mass of a calcium-free silicon compound and between 50% and 95% in mass of a calcium phosphate selected from: anhydrous dicalcium phosphate [monetite, CaHPO₄], dicalcium phosphate dihydrate [brushite, CaHPO₄·2H₂O], hydroxyapatite [Ca₁₀(PO₄)₆(OH)₂], amorphous calcium phosphate [Ca₃(PO₄)₂·nH₂O], and combinations thereof.

As shown, without limitation in the Examples and Figures provided as part of the present invention, materials of the present invention have, in most cases, a morphology that is predominantly crystalline and solubility profiles that contribute to the effective re-mineralization of teeth, occlusion of exposed dentinal tubules and consequently a reduction of dentinal hypersensitivity. Incorporation of divalent metallic ions (M) such as Mg⁺⁺, Zn⁺⁺, Ba⁺⁺ , Fe⁺⁺ , Sn⁺⁺ and Sr⁺⁺, as partial substitutions of Ca⁺⁺ in the calcium phosphates provides the means for effective release of these metallic ions that have also been found to contribute to tooth re-mineralization and reduction in the accumulation of bacterial plaque. Although the range of partial substitution of Ca⁺⁺ by divalent metallic ions (M) can be higher, in a preferred embodiment the range of substitution (x) of Ca⁺⁺ by divalent metallic ions (M) goes from cero (x=0), in the case of non-substituted calcium phosphates, up to 0.1 (x≤0.1) in the case were up to 10% of Ca⁺⁺ are partially substituted by the ion M. The different components of the re-mineralization materials of the present invention can have partial substitutions of Ca⁺⁺ by different metallic divalent ions (M) and it should be interpreted that the "x" hereby defines de substitution for each particular component and can therefore be different for the different components of a particular re-mineralization material. This is, for example, in a particular re-mineralization material the anhydrous dicalcium phosphate [monetite, Ca₁₋ₓMₓHPO₄], may have a partial substitution of Ca⁺⁺ by Zn++ defined by x=0.02 [monetite, Ca_{0.98}Zn_{0.02}HPO₄] while the hydroxyapatite in the same re-mineralization material [hydroxyapatite, Ca₁₀₋ₓMₓ(PO₄)₆(OH)₂] has a partial substitution of Ca⁺⁺ by Fe⁺⁺ defined by a x=0.05 [hydroxyapatite, Ca_{9.95}Fe_{0,05}(PO₄)₆(OH)₂], or no partial substitution and therefore the x=0,00 [hydroxyapatite, Ca₁₀(PO₄)₆(OH)₂]. Preferred divalent metallic ions (M) are magnesium, strontium, barium, iron, tin and/or zinc. Preferably, the calcium phosphates incorporating divalent metallic ions as partial substitutions of Ca⁺⁺ are present in at least 20% in total mass of the re-mineralizing material. These calcium phosphates incorporating divalent metallic ions (M) as partial substitutions of Ca⁺⁺ can therefore be in combination with other calcium phosphates to add up to between 50% and 95% in total mass of calcium phosphates in the re-mineralizing materials of the present invention. Therefore, in an embodiment of the present invention there is provided materials for tooth re-mineralization comprising between 1% and 40% in mass of calcium-free silicon compounds and between 50% and 95% in mass of calcium phosphates, and where at least 20% in total mass of the material is composed of calcium phosphates that incorporate divalent metallic ions (M) as partial substitutions of Ca⁺⁺ selected from: anhydrous dicalcium phosphate [monetite, Ca₁₋ₓMₓHPO₄], dicalcium phosphate dihydrate [brushite, Ca₁₋ₓMₓHPO₄·2H₂O], hydroxyapatite [Ca₁₀₋ₓMₓ(PO₄)₆(OH)₂], amorphous calcium phosphate [Ca₃₋ₓMₓ(PO₄)2·nH₂O], and combinations thereof, where 0<x≤0.1 and may be different for the different components of the re-mineralizing material, and where M is any divalent metallic ion that is not necessarily same for the different components of the re-mineralizing material.

In a realization of the invention, the re-mineralization materials comprises between 1% and 40% in mass calcium-free silicon compounds and between 50% and 95% in mass of calcium phosphates, and of these calcium phosphates hydroxyapatite where Ca++ is partially substituted by a divalent metallic ion (M) [Ca₁₀₋ₓMₓ(PO₄)₆(OH)₂], where 0<x≤0.1, makes up at least 20% in total mass of the re-mineralizing material.

A preferred embodiment incorporates the combination of calcium phosphates that do not have partial substitutions of Ca⁺⁺ selected from hydroxyapatite [Ca₁₀(PO₄)₆(OH)₂] and/or amorphous calcium phosphate [Ca₃(PO₄)₂·nH₂O] in combination with calcium phosphates incorporating divalent metallic ions (M) as partial substitutions of Ca⁺⁺ that represent at least 20% of the total weight of the re-mineralization material and are selected from anhydrous dicalcium phosphate [monetite, Ca₁₋ₓMₓHPO₄], dicalcium phosphate dihydrate [brushite, Ca₁₋ₓMₓHPO₄·2H₂O] where 0<x≤0.1, and may be different for the different components of the re-mineralizing material, and where M is a divalent metallic ion that is not necessarily same for the different components of the re-mineralizing material.

Examples incorporated as part of the present invention demonstrate the capacity of the materials of the present invention to occlude dentinal tubules and re-mineralize dentin, and the effectiveness of zinc in the re-mineralization and improvement of mechanical properties of treated tooth surface. Therefore, in a particular realization of the present invention, the divalent metallic ion (M) incorporated as a partial substitution of Ca⁺⁺ in the calcium phosphates is zinc. Therefore in an embodiment of the present invention there is provided materials for tooth re-mineralization comprising between 1% and 40% in mass of a calcium-free silicon compound and between 50% and 95% in mass of a calcium phosphates, and were at least 20% in total mass of the material is composed by calcium phosphates that incorporate Zn⁺⁺ as partial substitutions of Ca⁺⁺ and that are selected from: anhydrous dicalcium phosphate where Ca⁺⁺ is partially substituted by Zn⁺⁺ [Zn-monetite, Ca₁₋ₓZnₓHPO₄], dicalcium phosphate dihydrate where Ca⁺⁺ is partially substituted by Zn⁺⁺ [Zn-brushite Ca₁₋ₓZnₓHPO₄·2H₂O], amorphous calcium phosphate where Ca⁺⁺ is partially substituted by Zn⁺⁺ [Ca₃₋ₓZnₓ(PO₄)₂·nH₂O], hydroxyapatite where Ca⁺⁺ is partially substituted by Zn [Ca₁₀₋ₓZnₓ(PO₄)₆(OH)₂], amorphous calcium phosphate [Ca₃₋ₓZnₓ(PO₄)₂·nH₂O], and combinations thereof, where 0<x≤0.1 and may be different for the different components of the re-mineralizing material.

In a realization of the invention, the re-mineralization materials comprises between 1% and 40% in mass calcium-free silicion compounds and between 50% and 95% in mass of calcium phosphates, and of these calcium phosphates hydroxyapatite where Ca++ is partially substituted by Zn⁺⁺ [Ca₁₀₋ₓZnₓ(PO₄)₆(OH)₂],, where 0<x≤0.1, makes up at least 20% in total mass of the re-mineralizing material.

A preferred realization incorporates the combination of calcium phosphates that do not have partial substitutions of Ca⁺⁺, selected from hydroxyapatite [Ca₁₀(PO₄)₆(OH)₂] and/or amorphous calcium phosphate [Ca₃(PO₄)₂·nH₂O] in combination with calcium phosphates that incorporate Zn⁺⁺ as partial substitutions of Ca⁺⁺, representing at least 20% of the total weight of the re-mineralization material, selected from anhydrous dicalcium phosphate where Ca⁺⁺ is partially substituted by Zn⁺⁺ [Zn-monetite, Ca₁₋ₓZnₓHPO₄], dicalcium phosphate dihydrate where Ca⁺⁺ is partially substituted by Zn⁺⁺ [Zn-brushite Ca₁₋ₓZnₓHPO₄·2H₂O], and/or hydroxyapatite where Ca⁺⁺ is partially substituted by Zn [Ca₁₀₋ₓZnₓ (PO₄)₆(OH)₂], where 0<x≤0.1 and may be different for the different components of the re-mineralizing material.

Incorporation of the calcium-free silicon compounds in the materials of the present invention contributes to the release of silicon and improved capacity to occlude dentinal tubules and re-mineralize teeth. Preferred calcium-free silicon compounds are silicon oxide [SiO₂], silica gel [SiO₂·nH₂O], methasilicic acid [H₂SiO₃], orthosilicic acid [H₄SiO₄], silicic acid [H₆SiO₅], and combinations thereof. Therefore, according to an embodiment of the present invention there is provided materials for tooth re-mineralization comprising,
i. between 50% and 95% in mass of a calcium phosphates selected from: anhydrous dicalcium phosphate [monetite, CaHPO₄], dicalcium phosphate dihydrate [brushite, CaHPO₄·2H₂O], hydroxyapatite [Ca₁₀(PO₄)₆(OH)₂], amorphous calcium phosphate [Ca₃(PO₄)₂·nH₂O], dicalcium phosphate anhydrous [monetite, Ca₁₋ₓMₓHPO₄], dicalcium phosphate dihydrate [brushite Ca₁₋ₓMₓHPO₄·2H₂O], hydroxyapatite [Ca₁₀₋ₓMₓ (PO₄)₆(OH)₂], amorphous calcium phosphate [Ca₃₋ₓMₓ(PO₄)₂·nH₂O], and combinations thereof, where 40<x≤0.1, and may be different for the different components of the re-mineralizing material, and where M is any divalent metallic ion, not necessarily same for the different components of the re-mineralizing material, and
ii. between 1% and 40% in mass of a calcium-free silicon compound selected from: silicon oxide [SiO₂], silica gel [SiO_{2·}nH₂O], methasilicic acid [H₂SiO₃], orthosilicic acid [H₄SiO₄], silicic acid [H₆SiO₅] and combinations thereof.

Silica gel [SiO₂·nH₂O] is readily soluble and is a preferred calcium-free silicon compound to be combined with calcium phosphates. In an embodiment, dicalcium phosphates, monetite and brushite, and hydroxyapatite are preferred calcium phosphates to be combined with silica gel [SiO₂·nH₂O]. Furthermore, Zn⁺⁺ is a preferred partial substitution of Ca⁺⁺ in the calcium phosphates. Therefore, in an embodiment, of the present invention there is provided materials for tooth re-mineralization comprising,
i. between 50% and 95% of calcium phosphates of which at least 20% in total mass of the re-mineralization material comprises anhydrous dicalcium phosphate where Ca⁺⁺ is partially substituted by Zn [Zn-monetite, Ca₁₋ₓZnₓHPO₄] , dicalcium phosphate dihydrate where Ca⁺⁺ is partially substituted by Zn [Zn-brushite, Ca_{1- x}ZnₓHPO₄·2H₂O], hydroxyapatite where Ca⁺⁺ is partially substituted by Zn [Zn-hydroxyapatite, Ca₁₀₋ₓZnₓ (PO₄)₆(OH)₂], and combinations thereof, where 0<x≤0.1, and may be different for the different components of the re-mineralizing material, and
ii. between 1% and 40% in mass of silica gel [SiO₂·nH₂O].

Calcium phosphates and calcium-free silicon compounds of the present invention have a greater tooth re-mineralizing capacity when used in combination than when used separately. Calcium phosphates and calcium-fee silicon compounds may be combined in different amounts to obtain solid materials capable of tooth re-mineralization. Calcium-free silicon compounds may account between 1% and 40% of the total mass of the re-mineralizing material. In a preferred realization, calcium-free silicon compounds account for between 5% and 35% in mass of the re-mineralizing material.

It is envisaged that the re-mineralizing materials of the present invention may contain other components in addition to calcium phosphates and calcium-free silicon compounds. These additional materials include up to 20% of calcium silicates such as calcium metasilicate [CaSiO₃], dicalcium silicate [Ca₂SiO₄], tricalcium silicate [Ca₃SiO₅] and/or calcium silicate hydrate [C-S-H, with a 0.5 ≤ Ca:P ratio ≤ 2]. Of particular interest is the presence in the re-mineralizing material of between 1% and 20% in mass of calcium metasilicate [CaSiO₃]. Yet in a preferred realization, the material composition includes between 1% and 15% of calcium metasilicate [CaSiO₃]. Preferably, calcium metasilicate is wollastonite [CaSiO₃] and/or pseudo-wollastonite [CaSiO₃]. In a preferred embodiment the calcium-free silicon compound is silica gel, the preferred calcium phosphate is anhydrous dicalcium phosphate where Ca⁺⁺ is partially substituted by Zn [Zn-monetite, Ca₁₋ₓZnₓHPO₄] where 0≤x≤0.1, and the calcium silicate is calcium metasilicate [CaSiO₃]. So, in a realization of the present invention there is provided materials for tooth re-mineralization comprising,
i. between 1% and 40% in mass of silica gel [SiO₂·nH₂O], and
ii. between 50% and 95% of calcium phosphates of which at least 20% in total mass of the re-mineralization material comprises anhydrous dicalcium phosphate where Ca⁺⁺ is partially substituted by Zn [Zn-monetite, Ca₁₋ₓZnₓHPO₄] where 0<x≤0.1, and
iii. between 1% and 20% in mass of calcium metasilicate [CaSiO₃]

In yet another preferred embodiment of the re-mineralization materials the calcium-free silicon compound is silica gel, the preferred calcium phosphate is hydroxyapatite where Ca⁺⁺ is partially substituted by Zn [Zn-hydroxyapatite, Ca₁₀₋ₓZnₓ (PO₄)₆(OH)₂] and where 0≤x≤0.1, and the calcium silicate is calcium metasilicate [CaSiO₃]. So, in a realization of the present invention there is provided materials for tooth re-mineralization comprising,
i. between 50% and 95% of calcium phosphates of which at least 20% in total mass of the re-mineralization material comprises hydroxyapatite where Ca⁺⁺ is partially substituted by Zn [Zn-hydroxyapatite, Ca₁₀₋ₓZnₓ(PO₄)₆(OH)₂] and where 0≤x≤0.1, and
ii. between 1% and 40% in mass of silica gel [SiO₂·nH₂O], and
iii. between 1% and 20% in mass of calcium metasilicate [CaSiO₃]

It is anticipated that material may also comprise other calcium phosphates such as tricalcium phosphate [Ca₃(PO₄)₂], or silicon containing compounds such as bioactive glasses that that may further contribute to the capacity to re-mineralize exposed dentin.

Re-mineralizing materials of the present invention generally produced by hydraulic acid-base cementing reactions. The end-product of these acid-base chemical reactions is obtained from a rapid dissolution of calcium phosphates and silicates and re-precipitation to form a solid mixture formed by a combination of small crystals and an amorphous component. The amount of water is adjusted to permit continuous mixing of the reactants at the early stages of the reaction. This combination of amorphous and crystalline elements in the obtained materials provides with a great specific surface area and re-mineralizing activity on teeth. Therefore, in a realization, over 35% in mass of the re-mineralizing material is crystalline. In a preferred realization over 40% in mass of the re-mineralization material is crystalline. Yet in a preferred realization over 50% in mass of the re-mineralization material of the present invention is crystalline.

The reactants and conditions of the hydraulic acid-base cementing reactions (temperature, speed of addition, solid/liquid ratios) can determine the preferential formation of anhydrous dicalcium phosphate [monetite, CaHPO₄], hydrated dicalcium phosphate [brushite, CaHPO₄·2H₂O], hydroxyapatite [Ca₁₀(PO₄)₆(OH)₂] and/or amorphous calcium phosphate [Ca₃(PO₄)₂·nH₂O] and their equivalents incorporating different divalent metallic ions (M) as partial substitutions of Ca⁺⁺. The stoichiometry of the reactants and the stage of completeness of the hydraulic acid-base cementing reaction may be adjusted to obtain re-mineralizing materials with a basic, neutral or slightly acid pH. When necessary the resulting material may be washed to obtain a neutral pH although an acid pH may be preferred to obtain re-mineralizing materials that are more soluble in the oral cavity. Products of the hydraulic acid-base reaction may also be subsequently exposed to a basic environment, such as with NaOH or Na₂HPO₄, to favor the conversion of acidic calcium phosphates to basic calcium phosphates such as for example hydroxyapatite [Ca₁₀(PO₄)₆(OH)₂] or Zn-hydroxyapatite [Ca₁₀₋ₓZnₓ (PO₄)₆(OH)₂] where 0<x≤0.1.

Tooth re-mineralizing materials of the present invention obtained by hydraulic acid-base cementing reactions are solids formed by aggregates of small crystals and amorphous material. Re-mineralizing materials of the present invention are particularly easy to obtain with particle sizes appropriate for the formulation of re-mineralizing dentifrices. Comparatively, vitreous materials such as Novamin® have the disadvantage of requiring very high temperatures in the manufacture and have a hardness that makes them difficult to obtain the small particle sizes required for effective tooth re-mineralizing. Dentinal tubules have a diameter of 2-3 µm so particles with sizes bellow 2 µm are more effective in their occlusion. In a realization of the present invention the tooth re-mineralizing materials are in the form of solid particles with a size below about 20 µm, preferably less than about 10 µm, even more preferably less than about 5 µm. Ideally, the percentage of particles smaller than 2 µm is over 10%, preferably over 15%. Smaller particles have higher surface areas and solubility, and are more susceptible to dissolution and re-precipitation processes that contribute to effective re-mineralization of teeth.

Envisaged mode of action of the re-mineralizing materials of the present inventions is by physical obstruction of the dentinal tubules during brushing and by dissolution and precipitation of re-mineralizing material components as mineral, most likely apatite, on the tooth surface and interior of dentinal tubules. Particles of the re-mineralizing material of the present invention provide with the added advantage that during brushing particles penetrate deep into the dentinal tubules and other crevices, contributing to efficacious occlusion of dentinal tubules and tooth re-mineralization. Particles of the re-mineralizing materials of the present invention solubilize partially when exposed to aqueous environment such as that found in the oral cavity, liberating phosphate, silicate, calcium and, optionally, divalent metallic ions. These products of solubility contribute to the re-mineralization process through the formation of apatite on the surface of the re-mineralizing material and tooth surface. The tooth re-mineralizing materials of the present invention typically have a solubility between 5% and 25% in mass in the first hour of exposure to an aqueous environment.

Inclusion of particles of re-mineralizing materials of the present invention into dentifrices provides with an easy way for daily use in dental hygiene. It is established that dentifrices for daily use should not exceed the abrasiveness limit of 150 RDA units (Relative Dentin Abrasiveness). Re-mineralizing materials of the present invention are particularly suited for the formulation of re-mineralizing dentifrices for daily use as they are less hard and abrasive when compared to other re-mineralizing agents such as Novamin® bioglass. This allows for more frequent use and increased proportion of the re-mineralizing agent in the dentifrice.

Dentifrices incorporating the re-mineralizing materials of the present invention an be in the form of pastes, gels, powders, liquids, gums or other preparations for use in dental hygiene. In a realization the dentifrice is in the form of a powder mainly composed, that is in more than 90%, by the re-mineralizing materials of the present invention.

In a preferred realization, the dentifrice incorporating the re-mineralizing materials of the present invention is in the form of a toothpaste for daily use with toothbrushes. Preferred toothpastes are those that are mostly devoid of water and avoid dissolution of the re-mineralizing materials and undesirable pH changes that may occur in aqueous formulations. Non-aqueous compositions useful in the present invention preferably include a vehicle comprising an anhydrous humectant such as glycerol and polyethylene glycol. The amount of re-mineralizing material in the toothpaste may vary considerably. Comparatively, tooth-pastes for regular use such as GSK Sensodyne® Repair & Protect contain about 5% of a re-mineralizing agent such as Bioglass® 45S5 (Novamin®) and concentrations of about 20% of silica that serves as an abrasive and thickening agent. In the formulation of a dentifrice the re-mineralizing agents and additives used as abrasives and thickening agents may be substituted partially or totally by the re-mineralizing materials of the present invention. Therefore, toothpastes may be formulated in which the tooth re-mineralization agents completely or partially substitute the re-mineralizing agents such as Novamin® or were the tooth re-mineralizing materials of the present invention substitute the re-mineralizing agent and other additives such as silica. More so, the incorporation of divalent metal ions such as Zn in the re-mineralizing agents of the present invention also allow them to be used as a source of zinc instead of zinc citrate or zinc lactate present in about 2% in weight in some commercial tooth pastes such as Colgate® *Sensitive Multi Protection* and Oral-B *Pro Expert.* Therefore, in a realization of a dentifrice toothpaste the amount of re-mineralizing material of the present invention is between 0.25% and 40% in weight of a non-aqueous paste composition. Preferably, the amount of re-mineralizing material of the present invention is between 1% and 30% in weight. In yet a more preferred embodiment, the amount re-mineralizing material in the dentifrice is between 5% and 25% in weight. Therefore, according to a realization of the present invention there is provided dentifrices that comprise:
i. a non-aqueous vehicle, and
ii. between 0.25% and 40% in mass of a re-mineralizing material that comprises:
   a. Between 1% and 40% in mass of calcium-free silicon compounds and,
   b. Between 50% and 95% in mass of calcium phosphates.

In a preferred realization of the present invention incorporates dentifrices that comprise:
i.a non-aqueous vehicle, and
ii.between 0.25% and 40% in mass of a re-mineralizing material that comprises:
   a. Between 1% and 40% in mass of calcium-free silicon compounds and,
   b. Between 50% and 95% in mass of calcium phosphates of which at least 20% in total mass of the re-mineralization material is composed by calcium phosphates that incorporate Zn⁺⁺ as partial substitutions of Ca⁺⁺ and selected from: anhydrous dicalcium phosphate where Ca⁺⁺ is partially substituted by Zn⁺⁺ [Zn-monetite, Ca₁₋ₓZnₓHPO₄], dicalcium phosphate dihydrate where Ca⁺⁺ is partially substituted by Zn⁺⁺ [Zn-brushite Ca₁₋ₓZnₓHPO₄·2H₂O], hydroxyapatite where Ca⁺⁺ is partially substituted by Zn [Ca₁₀₋ₓZnₓ(PO₄)₆(OH)₂], amorphous calcium phosphate where Ca⁺⁺ is partially substituted by Zn⁺⁺ [Ca₃₋ₓZnₓ(PO₄)₂·nH₂O], and combinations of them, where 0<x≤0.1, and may be different for the different components of the re-mineralizing material.

Yet a preferred realization of the present invention incorporates dentifrices that comprise:
i.a non-aqueous vehicle, and
ii.between 0.25% and 40% in mass of a re-mineralizing material that comprises:
   a. Between 1% and 40% in mass of calcium-free silicon compounds and,
   b. Between 50% and 95% in mass of calcium phosphates of which at least 20% in total mass of the re-mineralization material is composed by anhydrous dicalcium phosphate where Ca⁺⁺ is partially substituted by Zn⁺⁺ [Zn-monetite, Ca₁₋ₓZnₓHPO₄] where 0<x≤0.1.

Dentifrice compositions containing the re-mineralization material of the present invention can incorporate other commonly used components such as abrasives, thickening agents, flavorings, sweetening agents and freshening agents. Of particular interest in the formulation of dentifrice compositions containing tooth re-mineralization materials of the present invention are sources of fluoride such as sodium fluoride, sodium monofluorophosphate or stannous fluoride.

Re-mineralizing materials of the present invention have the capacity to re-mineralize teeth upon exposure in the oral cavity for a period of less than 5 minutes. In preferred embodiment particles of the present invention have the capacity to re-mineralize teeth upon exposure in the oral cavity for a period of less than 3 minutes. Re-mineralization of teeth is a commonly used term broadly defined, but not bound, as the capacity to form an apatite on teeth. This apatite contributes to the occlusion of dentinal tubules, re-mineralization of dentin, re-mineralization of enamel and reduction of incidence of tooth sensitivity and caries.

As used herein, the term "comprising" is used in its broader sense as meaning "include", "contain" or "comprehend".

As used herein, the term "material for tooth re-mineralization", "re-mineralizing material" and "material for re-mineralization" are used indistinctively in their broadest sense referred to materials with the capacity to contribute to the mineralization of different structures in teeth.

As used herein, the term "dentrifice" includes any preparation intended for use in treatment or cleansing teeth, gums, periodontal regions, tooth pulp or root.

### DESCRIPTION OF FIGURES

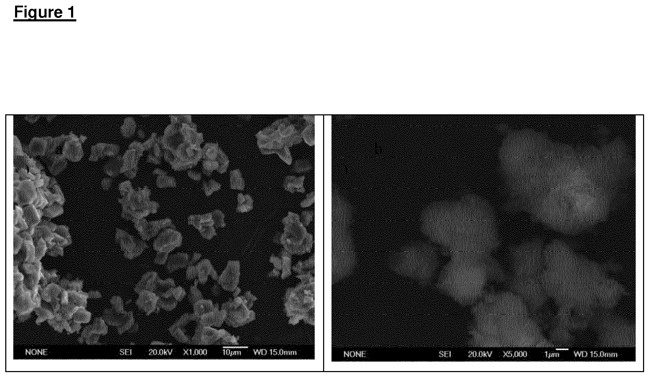

**Figure 1****:** Scanning electron microscopy (SEM) at x1,000 (Fig. 1a) and x5,000 (Fig. 1b) and (b) of re-mineralizing material of the present invention (designed as "F" in the Examples) containing calcium phosphates and calcium-free silicon compounds and produced by hydraulic cementing reactions that result in interlocking of crystals. Higher magnifications (Fig. 1b) show the micron size of the crystals that form the material of the present invention.

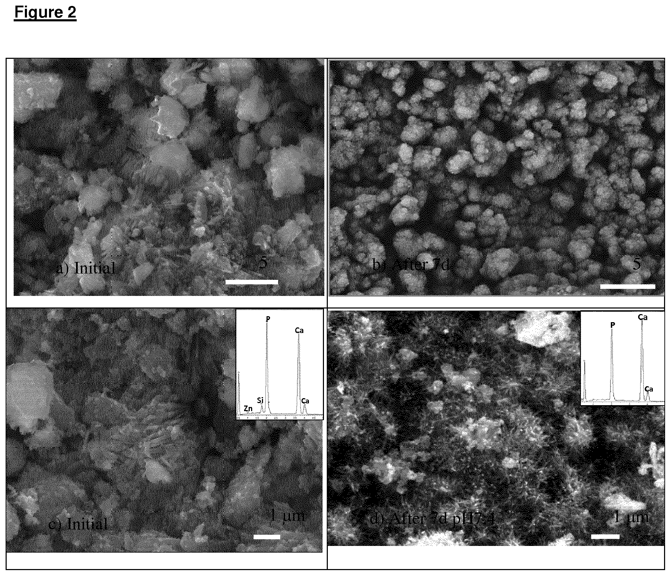

**Figure 2****:** Scanning electron microscopy (SEM) showing the predominantly crystalline morphology of the re-mineralization material (designed as "F in the Examples) at the start (Fig. 2a, and Fig. 2c) and after transformation after 7 days in artificial saliva (Fig. 2b) or at pH 7.4 in TRIS buffer (Fig. 2d). Newly formed crystals on the material surface indicate that the material is acting as a nucleation site for crystal growth. Changes in the P/Ca ratio measured by surface X-ray of the start material (Fig. 2c insert) and after 7 days in TRIS buffer at pH 7.4 (Fig. 2d insert) demonstrates that the material becomes covered with newly formed hydroxyapatite crystals.

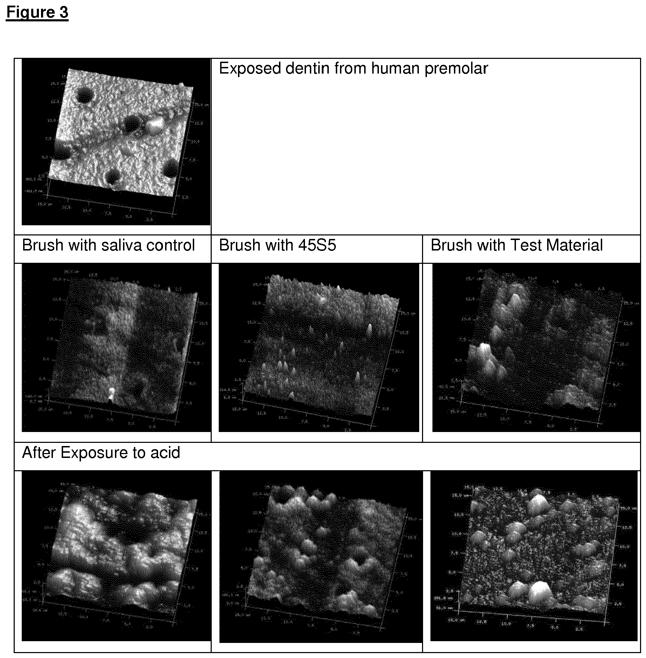

**Figure 3**: Atomic Force Spectroscopy (AFM) of a de-mineralized tooth with exposed dentin (Fig. 3a), after brushing with distilled water control (Fig. 3b), after brushing with 45S5 bioglass (Fig. 3c), and after brushing with a re-mineralizing material (designated as "F" in the Examples) of the present invention (Fig. 3d). Occlusion of dentinal tubes and formation of a mineral surface that is most significant for the re-mineralizing material "F" of the present invention. After acid treatment dentin treated with saliva control (Fig. 3e) showed a globular surface indicating de-mineralization and exposure of collagen, while dentin treated with 45S5 (Fig. 3f) and re-mineralizing material "F" (Fig. 3g) maintained a more heterogeneous surface with more inter-tubular roughness indicative of mineralization.

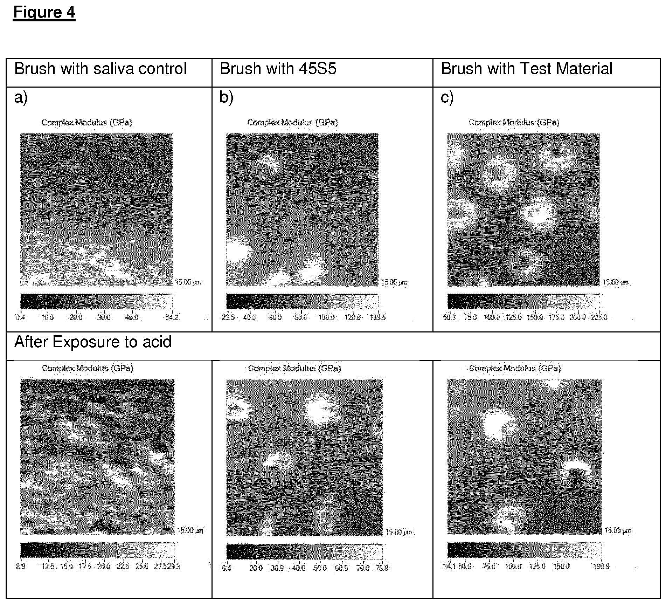

**Figure 4****:** Measurement of hardness (Complex Module in GPa) by means of serial nanoindentation of a demineralized dentin surface (15 x 15 micron) after brushing with distilled water control (Fig. 4a), brushing with 45S5 bioglass (Fig. 4b), and brushing with a re-mineralizing material (designated as "F" in the Examples) of the present invention (Fig. 4c). Occlusion of dentinal tubes and formation of a mineral surface resistant to indentation is more significant in dentin surfaces treated with re-mineralizing material "F". Furthermore, resistance to indentation after acid treatment of the control group (Fig. 4d) is inferior to those dentin surfaces treated with 45S5 (Fig. 4e) and much below dentin surfaces treated with re-mineralizing material "F" (Fig. 4f).

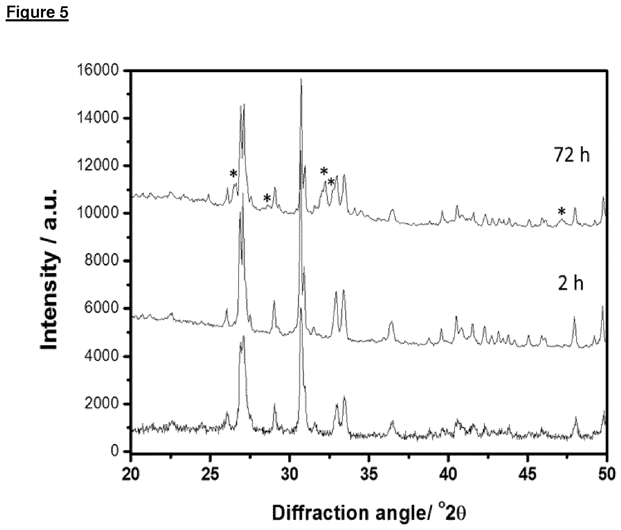

**Figure 5**: X-Ray diffraction pattern of a re-mineralizing material (designated as "D" in the Examples) of the present invention in its original state, 2 hours and 72 hours after immersion in water, showing a starting material which is significantly crystalline at the start and conversion of part of the material to hydroxyapatite (marked as * in the diffractogram).

### EXAMPLES

The present invention is further explained below by way of Examples that are presented as possible realizations and do not in any way pretend to be restrictive of the scope of the present invention.

**Example 1:** Composition, crystallinity and dissolution of different re-mineralization materials combining calcium phosphates and calcium-free silicon compounds.

By means of acid-base hydraulic reactions generally described in the review by Lawrence Chow [Next generation calcium phosphate-based biomaterials. Dent. Mater. J. 2009 Jan; 28(1): 1-10] different re-mineralization materials were synthesized ("A" to "H") and their composition determined as shows in the in the following Table.

| **Composition of different re-mineralization materials** | | | | | | | | |
|---|---|---|---|---|---|---|---|---|
| Material #: | "A" | "B" | "C" | "D" | "E" | "F" | "G" | "H" |
| *Monetite [CaHPO₄] | 42% | - | 20% | 51% | - | - | 60% | - |
| *Zn-Monetite, [Ca_{0.97}Zn_{0.03}H PO₄] | - | 70% | - | - | 50% | 57% | - | |
| *Brushite [CaHPO₄·2H₂O] | - | - | 65% | - | - | - | - | - |
| *Hydroxyapatite [Ca₁₀(PO₄)₆(OH)₂] | - | - | 10% | - | - | 25% | 20% | - |
| *Zn-Hydroxyapatite [Ca_{9.7}Zn_{0.3}(PO₄)₆(OH)₂] | | | | | | | | 40% |
| *Calcium metasilicate [CaSiO₃] | 20% | 10% | - | 6% | 5% | - | - | 33% |
| Amorphous calcium phosphate [Ca₃(PO₄)₂·nH₂O] | 11% | 10% | - | 12% | 15% | 11% | 8% | 15% |
| Silica gel [SiO₂·nH₂O], | 27% | 10% | 5% | 31% | 30% | 7% | 12% | 12% |
| Bioactive glass 70SiO₂-30CaO | - | - | - | - | 5% | - | - | - |
| Total crystalline phases | 62% | 80% | 95% | 57% | 55% | 82% | 75% | 73% |
| Dissolution % in mass in 1hr. | 10% | 8% | 22% | 7% | 10% | 6% | 7% | 10% |
| Resulting pH | 7.2 | 7.1 | 7.3 | 7.2 | 7.3 | 7.2 | 7.2 | 8.2 |

Composition of the crystalline phases, identified with an "*", were determined by quantitative X ray analysis. Composition estimates should be interpreted with an approximate ±5% error margin. Re-mineralization materials were crushed, sieved at 45 micron and their dissolution profile in artificial saliva compared to 45S5 bioglass. Re-mineralization materials of the present invention showed solubility ranging between 5% and 25% approximately compared to the solubility of 15% obtained for bioglass 45S5.

### Example 2: Dentin re-mineralization capacity of different re-mineralization materials combining calcium phosphates and calcium-free silicon compounds

Evaluation of the re-mineralizing capacity of materials of different composition was carried out on dentin discs obtained from human premolar teeth. Enamel was eliminated by sectioning and abrasion, elimination of the detritus from grinding and aperture of the tubules was carried out by subsequent treatment of the dentin surfaces with 0.5M EDTA.

The different re-mineralizing materials evaluated were mixed with water in a ratio between 2 and 5 ml/gr to obtain a paste that was applied to discs by brushing for one minute and then rinsed with water before immersion in artificial saliva solution at pH 7.2 and storage at 37ºC. The process was repeated twice a day for one week. After discs were exposed to an acid medium to simulate acidifying conditions in the oral cavity. Surface of treated discs, before and after the acid treatment, were evaluated by Raman spectroscopy to determine the presence of phosphate (Absorbance Units at 961 cm-1) and carbonate (Absorbance Units at 1070 cm-1) and nanoindentation (Hysitron Ti Premier, *Hysitron, Inc.,* USA) to determine the nano-hardness (Module in GPa) of the inter-tubular surface, peri-tubular surface and intra-tubular areas of the re-mineralized dentin. Results indicate the re-mineralizing capacity of all the evaluated compositions in comparison with the untreated control and increase hardness of dentin treated with some of the materials, "D" and "F" for example, when compared to dentin treated with 45S5. Increased hardness between and within the tubules indicates the efficacy of the re-mineralizing materials in occluding the dentinal tubules and re-mineralizing the tooth surface.

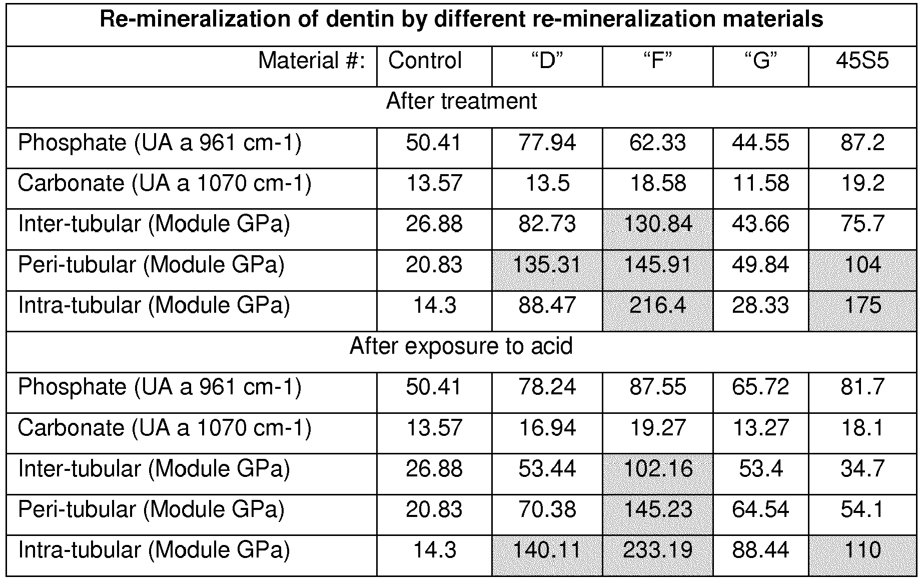

### Example 3: Formulation of toothpaste containing re-mineralizing materials comprising calcium phosphates and calcium-free silicon compounds of the present invention.

Re-mineralizing materials with the following estimated compositions "F⁺", G⁺" and "H" shown in the following Table were synthesized for the formulation of the toothpastes.

| **Re-mineralization materials used in formulation of toothpastes** | | | |
|---|---|---|---|
| Material #: | "F⁺" | "G⁺" | "H" |
| Zn-Monetite [Ca_{1-0,03}Zn_{0,03}HPO₄] | 57% | 29% | - |
| Hydroxyapatite [Ca₁₀(PO₄)₆(OH)₂] | 16% | 12% | - |
| Zn-Hydroxyapatite [Ca_{9.7}Zn_{0.3}(PO₄)₆(OH)₂] | - | - | 40% |
| Calcium Metasilicate [CaSiO₃] | - | 10% | 33% |
| Amorphous calcium phosphate [Ca₃(PO₄)₂·nH₂O] | 20% | 17% | 15% |
| Silica Gel [SiO₂·nH₂O] | 7% | 32% | 12% |

Synthesized materials were pulverized for 15 seconds in a ball mil and sieved at 45 microns. Powders obtained for each of the two re-mineralizing materials were mixed in a glass pestle and mortar with non-aqueous liquids for about 3 minutes to obtain a smooth paste with composition of the following Table.

| **Dentifrice formulation** | | |
|---|---|---|
| Component: | mass | % in mass |
| Re-mineralizing material | 40g | 23.26% |
| Glycerol | 100g | 58.14% |
| Polyethyleneglycol (PEG400) | 30g | 17.44% |
| Lauryl Sodium Sulphate | 2g | 1.16% |

As indicated in the following table, the percentage of particles with a particle size below 2 micron in the toothpastes formulated with these materials, "H" shown as an example, is considerably higher than that found in the commercial toothpaste Sensodyne® *Repair* & *Protect* (Glaxo Smithkline, UK) known to have 5% of Novamin and about 20% of silica. This was found in the formulated toothpastes and in the saliva after normal tooth brushing. Very similar values found for toothpastes formulated with "F⁺", "G⁺" or "H". Presence of greater number of particles with a particle size below 2 micron contributes to the efficacious occlusion of the dentinal tubules known to have a diameter of 2 to 3 microns (µm).

| **Evaluation of particle size of re-mineralizing material in a toothpaste** | | | | | |
|---|---|---|---|---|---|
| % particles in Volume: | <2µm | <5µm | < 10 µm | < 20µm | < 100µm |
| Powder | ≈ 20% | ≈ 60% | ≈ 86% | ≈97% | ≈100% |
| Toothpaste | ≈ 16% | ≈ 50% | ≈ 74% | ≈91% | ≈100% |
| Saliva after tooth brushing | ≈ 18% | ≈ 55% | ≈ 85% | ≈97% | ≈100% |
| Toothpaste after storage for 7 days | ≈ 16% | ≈ 50% | ≈ 74% | ≈91% | ≈100% |
| Sensodyne® | ≈ 1% | ≈ 10% | ≈ 23% | ≈53% | ≈100% |

The three toothpastes were evaluated in mouth by gentle tooth brushing for 1-2 minutes with an approximate volume of 100 µl. pH of the saliva was found not to change from the baseline value of 7 recorded prior to tooth brushing. Neither of the re-mineralizing materials or the formulations appeared to have taste and the user reported a positive feeling after use. Preliminary *in vitro* tests demonstrated the efficacy of these pastes in the occlusion of dentinal tubules and tooth re-mineralization.

## Claims

1. Material for tooth re-mineralization comprising between 50% and 95% in mass of calcium phosphates and between 1% and 40% in mass of calcium-free silicon compounds.

2. Material for re-mineralization of teeth according to claim 1 where the calcium phosphates are selected from the list comprising: dicalcium phosphate anhydrous [monetite, CaHPO₄], dicalcium phosphate dihydrate [brushite CaHPO₄·2H₂O], hydroxyapatite [Ca(PO₄)₆(OH)₂], amorphous calcium phosphate [Ca(PO₄)₂·nH₂O], dicalcium phosphate anhydrous [monetite, Ca₁₋ₓMₓHPO₄], dicalcium phosphate dihydrate [brushite Ca₁₋ₓMₓHPO₄·2H₂O], hydroxyapatite [Ca₁₀₋ₓMₓ(PO₄)₆(OH)₂], amorphous calcium phosphate [Ca₃₋ₓMₓ(PO₄)₂·nH₂O], and combinations thereof, where 0<x≤0.1 and may be different for the different components of the re-mineralizing material, and where M is any divalent metallic ion, that is not necessarily same for the different components of the re-mineralizing material.

3. Material for re-mineralization of teeth according to any of claims 1 or 2 in which at least 20% in mass of the material is comprised by calcium phosphates selected from dicalcium phosphate anhydrous [monetite, Ca₁₋ₓMₓHPO₄], dicalcium phosphate dihydrate [brushite Ca₁₋ₓMₓHPO₄·2H₂O], hydroxyapatite [Ca₁₀₋ₓMₓ(PO₄)₆(OH)₂], amorphous calcium phosphate [Ca₃₋ₓMₓ(PO₄)₂·nH₂O], where 0<x≤0.1 and may be different for the different components of the re-mineralizing material, and where M is any divalent metallic ion, that is not necessarily same for the different components of the re-mineralizing material.

4. Material for re-mineralization of teeth according to any of claims 2 or 3 in which M of one or more of the calcium phosphates is zinc (Zn).

5. Material for re-mineralization of teeth according to any of claims 1 to 4 in which the calcium-free silicon compounds are selected from the list that comprises silicon oxide [SiO₂], silica gel [SiO2·nH2O], methasilicic acid [H₂SiO₃], orthosilicic acid [H₄SiO₄], silicic acid [H₆SiO₅] and combinations thereof.

6. Material for re-mineralization of teeth according to any of claims 1 to 5 in which at least 20% in mass of the material for tooth re-mineralization is comprised by calcium phosphates selected from anhydrous dicalcium phosphate where Ca⁺⁺ is partially substituted by Zn [Zn-monetite, Ca₁₋ₓZnₓHPO₄], dicalcium phosphate dihydrate where Ca⁺⁺ is partially substituted by Zn [Zn-brushite, Ca₁₋ₓZnₓHPO₄·2H₂O], hydroxyapatite where Ca⁺⁺ is partially substituted by Zn [Zn-hydroxyapatite, Ca₁₀₋ₓZnₓ(PO₄)₆(OH)₂], and combinations thereof, where 0<x≤0.1, and may be different for the different components of the re-mineralizing material, and the calcium-free silicon compound is silica gel [SiO₂·nH₂O].

7. Material for re-mineralization of teeth according to any of claims 1 to 6 that incorporates between 1% and 20% in mass of calcium metasilicate [CaSiO3].

8. Material for re-mineralization of teeth according to any of claims 1 to 6 that is crystalline in more than 35% in mass.

9. Material for re-mineralization of teeth according to any of claims 1 to 8 with a solubility between 5% and 25% in mass within the first hour of exposure to an aqueous medium.

10. Material for re-mineralization of teeth according to any of claims 1 to 8 in the form of solid particles with an average size below 20 microns.

11. Dentifrice in the form of paste in which the material for the re-mineralization of teeth of the present invention of any of claims 1 to 10 is present between 0.25% and 40% in weight in an non-aqueous composition.

12. Dentifrice according to claim 11 in which the material for the re-mineralization of teeth comprises between 1% and 40% in mass of silica gel [SiO₂·nH₂O], and between 50% and 95% in mass of calcium phosphates of which at least 20% in total mass of the re-mineralization material is composed by anhydrous dicalcium phosphate where Ca⁺⁺ is partially substituted by Zn⁺⁺ [Zn-monetite, Ca₁₋ₓZnₓHPO₄], dicalcium phosphate dihydrate where Ca⁺⁺ is partially substituted by Zn⁺⁺ [Zn-brushite Ca₁₋ₓZnₓHPO₄·2H₂O], hydroxyapatite where Ca⁺⁺ is partially substituted by Zn [Ca₁₀₋ₓZnₓ (PO₄)₆(OH)₂], amorphous calcium phosphate where Ca⁺⁺ is partially substituted by Zn⁺⁺ [Ca₃₋ₓZnₓ(PO₄)₂·nH₂O], and combinations of them, where 0<x≤0.1, and may be different for the different components of the re-mineralizing material.

13. Dentifrice comprised in more than 95% by the material for tooth re-mineralization of any of claims 1 to 10.

14. Use of a material for tooth re-mineralization according to any of claims 1 to 10 for the manufacture of a cosmetic or pharmaceutical composition for tooth re-mineralization, prevention of caries and tooth decay, re-mineralization of dentin and enamel, occlusion of dentinal tubules and treatment of dentin hypersensitivity.

15. Use of a material for tooth re-mineralization according to any of claims 1 to 10 for the manufacture of a dentifrice or the dentifrice according to any of claims 11 to 13 for the re-mineralization of teeth.
